# EUROPÄISCHE PATENTSCHRIFT

(11) **EP 0 579 848 B1**
(45) Veröffentlichungstag und Bekanntmachung des Hinweises auf die Patenterteilung: **04.10.1995**
(21) Anmeldenummer: 92112381.6
(22) Anmeldetag: 20.07.1992
(51) Int. Cl.: H05G 1/30, H05G 1/70, G01N 23/04

(54) **Prüfanlage für Gegenstände**
Testing system for objects
Appareil d'inspection d'objets

(43) Veröffentlichungstag der Anmeldung: 26.01.1994
(73) Patentinhaber: Heimann Systems GmbH, D-65020 Wiesbaden (DE)
(72) Erfinder: Henkel, Rainer, Dipl.-Ing., W-6200 Wiesbaden (DE); Zimmer, Manfred, Dipl.-Ing., W-6500 Mainz (DE); Bermbach, Rainer, Dr.-Ing., W-6500 Mainz (DE)

(56) Entgegenhaltungen:
- DE-A- 3 708 843
- DE-A- 4 023 414
- US-A- 4 080 536
- US-A- 5 091 924
- US-A- 5 107 528
- PATENT ABSTRACTS OF JAPAN vol. 7, no. 241 (P-232)(1386) 26. Oktober 1983 & JP-58 127 153

## Beschreibung

Aus der DE-A 40 23 414 ist eine Vorrichtung zum Durchstrahlen von Gegenständen mit fächerförmiger Strahlung bekannt, die beispielsweise an Flughäfen eingesetzt wird, um den Inhalt von Gegenständen auf Bomben, Waffen und Schmuggelgut zu durchsuchen. Hierzu weist diese Vorrichtung ein erstes und ein zweites bildgebendes System mit jeweils einem Strahlensender und einem diesem zugeordneten Strahlenempfänger auf. Ein Gegenstand kann somit aus unterschiedlichen Richtungen durchstrahlt werden. Die dem Durchstrahlungsbild entsprechenden Signale der Strahlenempfänger werden einer Bildverarteitungsvorrichtung mit einem Rechner zugeführt der aus den Signalen ein sichtbares Bild auf einem Monitor des bildgebenden Systems erstellt.

Aus der DE-A 37 08 843 und der US-A 5,107,528 sind Prüfanlagen für Gegenstände mit einem ersten und einem zweiten bildgebenden System zum jeweiligen Erzeugen eines Durchstrahlungsbildes des Gegenstandes als sichtbares Bild auf einem Monitor bekannt, die eine gemeinsame Einheit zum Steuern des ersten und zweiten bildgebenden Systems enthalten.

Der Erfindung liegt die Aufgabe zugrunde, eine Prüfanlage für Gegenstände so auszuführen, daß die Bedienung gegenüber dem Stand der Technik weiter vereinfacht und verbessert ist.

Diese Aufgabe wird gelöst durch eine Prüfanlage für Gegenstände mit einem ersten und einem zweiten bildgebenden System zum jeweiligen Erzeugen eines Durchstrahlungsbildes des Gegenstandes als sichtbares Bild auf einem Monitor, mit einer gemeinsamen Bedieneinheit zum Steuern des ersten und zweiten bildgebenden Systems, wobei auf dem Monitor des ersten bildgebenden Systems eine erste Ansicht und auf dem Monitor des zweiten bildgebenden Systems eine zweite Ansicht des Gegenstandes darstellbar ist, und wobei über die Bedieneinheit eine gleichzeitige Manipulation der Darstellungen bewirkbar ist. Die Manipulation der Darstellungen muß somit nicht mehr gesondert bei jedem bildgebenden System vorgenommen werden. Diese Ausführung ist insbesondere dann vorteilhaft, wenn über die Bedieneinheit eine gleichzeitige Verschiebung der Darstellungen auf den Monitoren bewirkt werden soll. Es ist somit möglich, auf dem Monitor des ersten bildgebenden Systems eine Draufsicht und auf dem Monitor des zweiten bildgebenden Systems eine Seitenansicht des Gegenstandes darzstellen, wobei dann die Darstellungen synchron zueinander auf den Bildschirmen der Monitore verschoben werden können.

Vorzugsweise sind die bildgebenden Systeme einzeln oder gemeinsam von der Bedieneinheit steuerbar und werden Signale der Bedieneinheit über einen Verteiler dem ersten und/oder zweiten bildgebenden System zugeführt. Der Verteiler lenkt somit Signale der Bedieneinheit entweder zu dem ersten und/oder zu dem zweiten bildgebenden System.

In weiterer vorteilhafter Ausgestaltung ist vorgesehen, daß über die Bedieneinheit vorgebbar ist, ob eine Recheneinheit des ersten oder zweiten bildgebenden Systems die Funktion des Masters zum Übertragen von Daten und Signalen von dem betreffenden bildgebenden System zum anderen bildgebenden System oder die Funktion des Slaves erfüllt, und daß der Grad der gleichzeitigen Manupulation bzw. der Kopplung zwischen Master und Slave vorgebbar ist. Die bildgebenden Systeme können somit miteinander kommunizieren.

Die Prüfanlage für Gegenstände ist des weiteren kostengünstig herstellbar, da auf eine zweite Bedieneinheit verzichtet werden kann.

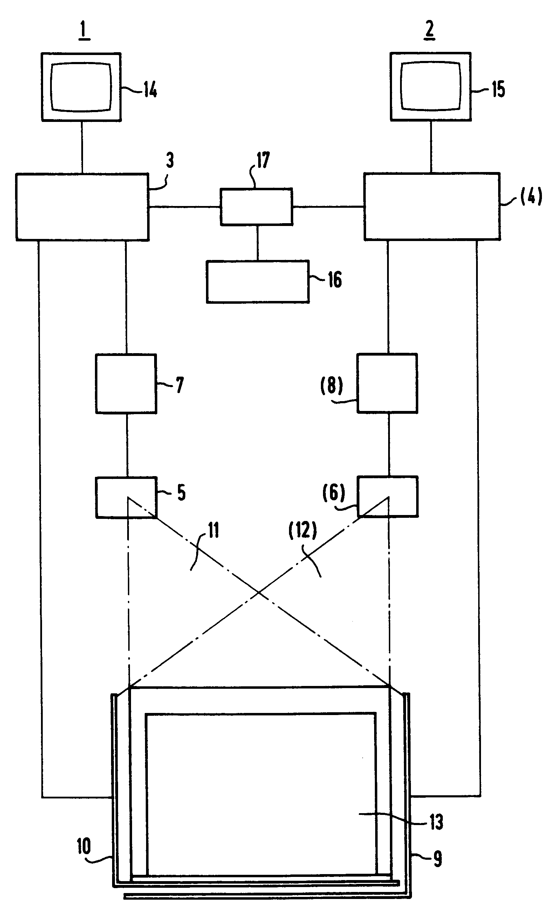

Weitere Vorteile und Einzelheiten der Erfindung ergeben sich aus der nachfolgenden Beschreibung eines Ausführungsbeispieles einer Prüfanlage für Gegenstände nach der Erfindung anhand der Zeichnung.

In der Figur ist in prinzipieller Weise eine Prüfanlage für Gegenstände, beispielsweise Fracht, Behälter, Container- und/oder Fahrzeuge gezeigt, die ein erstes und zweites bildgebendes System 1, 2 aufweist. Die bildgebenden Systeme 1, 2 besitzen gleiche Funktionselemente, wobei die Funktionselemente des zweiten bildgebenden Systems 2 nachfolgend mit in Klammern gesetzten Bezugszahlen gekennzeichnet sind. So besitzt das bildgebende System 1, (2) eine Recheneinheit 3, (4) zum Steuern einer Aufnahmeeinheit, die einen Strahlensender 5, (6) aufweist, der von einem Generator 7, (8) mit Energie versorgbar ist. Dem Strahlensender 5, (6) ist ein Strahlenempfänger 9, (10) zugeordnet, der das Strahlenbündel 11, (12) des Strahlensenders 5, (6), nach dem dieses einen Gegenstand als Untersuchungsobjekt, beispielsweise einen Container 13, durchdrungen hat, als Durchstrahlungsbild des Containers 13 empfängt. Dieses Durchstrahlungsbild wird vom Strahlenempfänger 9, (10) in elektrische Signale gewandelt, die der Recheneinheit 3, (4) zugeführt werden. Die Recheneinheit 3, (4) berechnet aus diesen Signalen ein sichtbares Bild, welches auf einem Monitor 14 (15) darstellbar ist.

Zur besseren Erkennbarkeit von Gegenständen im Container 13 wird dieser vorzugsweise aus unterschiedlichen Richtungen durchstrahlt, so daß sich auf den Monitoren 14, (15) unterschiedliche Ansichten des Containers 13 als Darstellungen zeigen.

Die gezeigte Prüfanlage für Gegenstände besitzt ferner eine gemeinsame Bedieneinheit 16, deren Signale der Recheneinheit 3, (4) zuführbar sind. Über die Bedieneinheit 16 können die Aufnahmeparameter des Strahlensenders 5, (6), der beispielsweise als Röntgenröhre ausgeführt ist, (kv, mA, t) für jedes Aufnahmesystem einzeln oder gemeinsam über eine Tastatur eingestellt werden. Zudem ist es möglich, eine gleichzeitige Manipulation der Darstellungen auf den Monitoren 14 (15) zu bewirken. So kann beispielsweise der Kontrast und/oder die Helligkeit verändert werden. Vorzugsweise kann über die Bedieneinheit 16 aber eine gleichzeitige Verschiebung der Darstellungen des Containers 13 auf den Monitoren 14, (15) bewirkt werden, so daß beispielsweise eine Seiten- und eine Draufsicht des Containers 13 synchron auf dem Bildschirm der Monitore 14, (15) verschoben werden können.

Zwischen die Bedieneinheit 16 und die Recheneinheit 3, (4) ist ein Verteiler 17 geschaltet, der die Signale der Bedieneinheit 16 der Recheneinheit 3 und/oder der Recheneinheit (4) zuführt. Es ist somit möglich, Signale der Bedieneinheit 16 beispielsweise der Recheneinheit 3 zuzuführen, die diese Signale verarbeitet. Die verarbeiteten Signale können dann zur Steuerung des zweiten bildgebenden Systems (2) über den Verteiler 17 der Recheneinheit (4) zugeführt werden. Die Recheneinheit 3 ist somit Master und die Recheneinheit (4) somit Slave. Selbstverständlich kann im Rahmen der Erfindung auch die Recheneinheit (4) die Funktion des Masters und die Recheneinheit 3 die Funktion des Slaves übernehmen. Durch Funktionselemente der Bedieneinheit 16 kann vorbestimmt werden, ob Signale der Bedieneinheit 16 nur der Recheneinheit 3 oder auch der Recheneinheit (4) zugeführt werden, so daß Rechenoperationen und Manipulationen der Darstellungen einzeln oder gemeinsam von den Recheneinheiten 3, (4) für jedes bildgebende System 1, 2 ausgeführt werden. Beispielsweise kann den der Strahlenabsorption zugeordneten Grauwerten eine Farbe zugeordnet werden. Über die Bedieneinheit 16 ist vorgebbar, ob beide Darstellungen in Farbe oder ob eine Darstellung in Grauwerten und die andere in Farbe auf dem Bildschirm der Monitore 14, (15) angezeigt wird. Die Darstellungen können einzeln oder auch synchron auf dem Bildschirm der Monitore 14, (15) verschoben werden. Ferner ist steuerbar, ob beispielsweise der Kontrast und/oder die Helligkeit der Darstellungen einzeln oder gemeinsam in gleicher oder unterschiedlicher Weise verändert werden soll. Über die Bedieneinheit 16 ist also der Grad der gleichzeitigen Manipulation bzw. der Kopplung zwischen Master und Slaves vorgebbar.

Weil die erfindungsgemäße Prüfanlage für Gegenstände mit zwei Monitoren 14, (15) versehen ist, ist es vorteilhaft, wenn diesen synchronisierte Videosignale zugeführt werden, um Interferenzstörungen und Flimmererscheinungen auf den Bildschirmen zu vermeiden.

In weiterer Ausgestaltung der erfindungsgemäßen Prüfanlage für Gegenstände können Mittel zur Erzeugung eines Fensters zur Auswertung der Darstellung auf dem bzw. den Bildschirm(en) eines Monitores oder beider Monitore 14, (15) vorgesehen sein, wobei das Fenster bzw. die Fenster über die Bedieneinheit (16) steuerbar ist bzw. sind. Selbstverständlich können auch mehrere Fenster zur Auswertung einer Darstellung auf dem Bildschirm des Monitors gesteuert werden.

Es sei noch erwähnt, daß die Bedieneinheit 16 zur Eingabe von Aufnahmeparametern, zur Steuerung der bildgebenden Systeme und zur Manipulation der Darstellungen nicht nur eine Tastatur, sondern auch z.B. eine Maus, ein Joy-Stick, ein Trackball oder beliebige Kombinationen davon aufweisen kann, wodurch die Bedienung vereinfacht ist.

## Patentansprüche

1. Prüfanlage für Gegenstände mit einem ersten und einem zweiten bildgebenden System (1, 2), zum jeweiligen Erzeugen eines Durchstahlungsbildes des Gegenstandes (13) als sichtbares Bild auf einem Monitor (14, 15), mit einer gemeinsamen Bedieneinheit (16) zum Steuern des ersten und zweiten bildgebenden Systems (1,2), wobei auf dem Monitor (14) des ersten bildgebenden Systems (1) eine erste Ansicht und auf dem Monitor (15) des zweiten bildgebenden Systems (2) eine zweite Ansicht des Gegenstandes (13) darstellbar ist, **dadurch gekennzeichnet,** daß über die Bedieneinheit (16) eine gleichzeitige Manipulation der Darstellungen bewirkbar ist.

2. Prüfanlagen für Gegenstände nach Anspruch 1, **dadurch gekennzeichnet**, daß die bildgebenden Systeme (1, 2) einzeln oder gemeinsam von der Bedieneinheit (16) ansteuerbar sind, und daß Signale der Bedieneinheit (16) über einen Verteiler (17), dem ersten und/oder zweiten bildgebenden System (1, 2) zugeführt werden.

3. Prüfanlage für Gegenstände nach einem der Ansprüche 1 oder 2, **dadurch gekennzeichnet,** daß über die Bedieneinheit (16) vorgebbar ist, ob eine Recheneinheit (3, 4) des ersten oder zweiten bildgebenden Systems (1, 2) die Funktion des Masters zum Übertragen von Daten oder Signalen von dem betreffenden bildgebenden Systems zum anderen bildgebenden System oder die Funktion des Slaves erfüllt, und daß der Grad der gleichzeitigen Manipulation bzw. der Kopplung zwischen Master und Slave vorgebbar ist.

4. Prüfanlage für Gegenstände nach einem der Ansprüche 1 bis 3, **dadurch gekennzeichnet,** daß über die Bedieneinheit (16) eine gleichzeitige Verschiebung der Darstellungen auf den Monitoren (14, 15) bewirkt werden kann.

5. Prüfanlage für Gegenstände nach einem der Ansprüche 1 bis 4, **dadurch gekennzeichnet**, daß über die Bedieneinheit (16) wenigstens ein Fenster zur Auswertung der Darstellung auf dem Bildschirm wenigstens eines Monitors (14, 15) steuerbar ist.

## Claims

1. Testing installation for objects, with a first and a second imaging system (1,2) for the production of a radiation image of the object (13) as a visible image on monitors (14,15) respectively, with a common operating unit (16) for controlling the first and second imaging systems (1,2), a first view of the object (13) being producible on the monitor (14) of the first imaging system (1) and a second view on the monitor (15) of the second imaging system (2), characterised by the fact that the portrayal can be manipulated simultaneously by means of the operating unit (16).

2. Testing installation for objects, in accordance with Claim 1, characterised by the fact that the imaging systems (1,2) can be activated separately or jointly by the operating unit (16) and that signals from the operating unit (16) are conveyed via a distributor (17) to the first and/or second imaging system (1,2).

3. Testing installation for objects, in accordance with either of Claims 1, 2, characterised by the fact that it can be determined in advance by means of the operating unit (16) whether a computing unit (3,4) of the first or second imaging system (1,2) will fulfil the master function for the transmission of data or signals from the relevant imaging system to the other imaging system, or fulfils the slave function, and that the degree of simultaneous manipulation or coupling between master and slave is predeterminable.

4. Testing installation for objects, in accordance with any one of Claims 1 to 3, characterised by the fact that the operating unit (16) can be used to effect a simultaneous displacement of the portrayals on the monitors (14,15).

5. Testing installation for objects, in accordance with any one of Claims 1 to 4, characterised by the fact that the operating unit (16) can be used to control the portrayal of at least one monitor (14,15) on the screen.

## Revendications

1. Appareil de détection d'objets comportant un premier et un deuxième système générateur d'images pour produire chacun une image radioscopique de l'objet (13) en tant qu'image visible sur un moniteur (14, 15), ainsi qu'une unité de commande commune (16) pour commander le premier et le deuxième générateur d'images (1, 2), une première vue de l'objet (13) pouvant être représentée sur le moniteur (14) du premier système générateur d'images (1), et une deuxième vue de l'objet (13) pouvant être représentée sur le moniteur (15) du deuxième système générateur d'images (2), caractérisé en ce qu'on peut effectuer par l'intermédiaire de l'unité de commande (16) une manipulation simultanée des images.

2. Appareil d'inspection d'objets selon la revendication 1, caractérisé en ce que les systèmes générateurs d'images (1, 2) peuvent être commandés individuellement ou ensemble par l'unité de commande (16) et en ce que des signaux de l'unité de commande (16) peuvent être amenés par l'intermédiaire d'un distributeur (17) au premier et/ou au deuxième système générateur d'images (1, 2).

3. Appareil d'inspection d'objets selon l'une des revendications 1 ou 2, caractérisé en ce qu'on peut prédéfinir par l'intermédiaire de l'unité de commande (16) si un ordinateur (3, 4) du premier ou du deuxième système générateur d'images (1, 2) assure la fonction de maître pour transmettre des données ou des signaux du système générateur d'images concerné à l'autre système générateur d'images, ou s'il assure la fonction d'ordinateur asservi, et en ce qu'on peut prédéfinir le degré de manipulation et/ou de couplage simultané entre l'ordinateur maître et l'ordinateur asservi.

4. Appareil d'inspection d'objets selon l'une des revendications 1 à 3, caractérisé en ce qu'on peut effectuer par l'intermédiaire de l'unité de commande (16) un déplacement simultané des images sur les moniteurs (14, 15).

5. Appareil d'inspection d'objets selon l'une des revendications 1 à 4 caractérisé en ce qu'on peut commander, par l'intermédiaire de l'unité de commande (16) au moins une fenêtre pour évaluer l'image sur l'écran d'au moins un moniteur (14, 15).
